# EUROPÄISCHE PATENTSCHRIFT

(11) **EP 1 867 984 B1**
(45) Veröffentlichungstag und Bekanntmachung des Hinweises auf die Patenterteilung: **07.01.2009**
(21) Anmeldenummer: 06115455.5
(22) Anmeldetag: 14.06.2006
(51) Int. Cl.: G01N 31/00, G01N 33/18

(54) **Testkit zur Bestimmung des organisch gebundenen Kohlenstoffs in einer mit Partikeln belasteten Probe**
Testkit for determining total organic carbon in a sample polluted with particles
Kit de test pour déterminer la teneur totale en carbone organique dans un échantillon pollué par des particules

(43) Veröffentlichungstag der Anmeldung: 19.12.2007
(73) Patentinhaber: Hach Lange GmbH, 14163 Berlin (DE)
(72) Erfinder: Lundgreen, Ulrich, 33330 Gütersloh (DE); Lenhard, Markus, 41751 Viersen (DE); Geick, Klaus, 41516 Grevenbroich (DE); Ketterer, Klaus, 40667 Meerbusch (DE); Rieger, Claudia, 40227 Düsseldorf (DE); Farjam, Aria, 6291 HD, Vaals (NL)
(74) Vertreter: Fitzner, Uwe

(56) Entgegenhaltungen:
- WO-A-00/75653
- US-A1- 2005 282 286
- HUANG K C ET AL: "Kinetics of heat-assisted persulfate oxidation of methyl tert-butyl ether (MTBE)" CHEMOSPHERE, PERGAMON PRESS, OXFORD, GB, Bd. 49, Nr. 4, Dezember 2002 (2002-12), Seiten 413-420, XP009071578 ISSN: 0045-6535
- ANONYMUS: "Sicherheitsdatenblatt"[Online] 9. März 2004 (2004-03-09), Seiten 1-6, XP002396515 Gefunden im Internet: URL:http://www.merck.de/servlet/PB/menu/10 07900/index.html> [gefunden am 2006-08-28]
- PATENT ABSTRACTS OF JAPAN Bd. 007, Nr. 212 (P-224), 20. September 1983 (1983-09-20) -& JP 58 106459 A (TOKYO SHIBAURA DENKI KK), 24. Juni 1983 (1983-06-24)
- BISUTTI I ET AL: "Determination of total organic carbon - an overview of current methods" TRAC, TRENDS IN ANALYTICAL CHEMISTRY, ELSEVIER, AMSTERDAM, NL, Bd. 23, Nr. 10-11, November 2004 (2004-11), Seiten 716-726, XP004654696 ISSN: 0165-9936
- SPYRES G ET AL: "Determination of dissolved organic carbon in seawater using high temperature catalytic oxidation techniques" TRAC, TRENDS IN ANALYTICAL CHEMISTRY, ELSEVIER, AMSTERDAM, NL, Bd. 19, Nr. 8, August 2000 (2000-08), Seiten 498-506, XP004213808 ISSN: 0165-9936
- HUANG ET AL: "Degradation of volatile organic compounds with thermally activated persulfate oxidation" CHEMOSPHERE, PERGAMON PRESS, OXFORD, GB, Bd. 61, Nr. 4, Oktober 2005 (2005-10), Seiten 551-560, XP005098106 ISSN: 0045-6535
- DATABASE WPI Thomson Scientific, London, GB; AN 1992-173777 & SU 1 658 045 A 24. Januar 1989 (1989-01-24)
- DATABASE WPI Thomson Scientific, London, GB; AN 1989-201297 & JP 01 021355 A 23. Juni 1991 (1991-06-23)

## Beschreibung

Die vorliegende Erfindung betrifft ein Drukschrifft .

In der analytischen Chemie sind solche Methoden von besonderer Bedeutung, bei denen der zu bestimmende Parameter durch Überführung in die Gasform selektiv aus dem Probengemisch abgetrennt wird. Hierbei kann nach Überführung in die Gasphase die direkte Bestimmung beispielsweise mittels Gaschromatographie, Atomadsorption, IR- oder Chemolumineszenzspektroskopie durchgeführt werden. Daneben gibt es auch weitere Möglichkeiten der Bestimmung z.B. die Konduktometrie, die Coulomentrie, die Potentiometrie, der Gasvolumentrie, der acidimetrischen Maßanalyse, der manometrischen Messung, der jodometrischen Maßanalyse und der Photometrie.

In der Wasseranalytik beruhen alle bekannten analytischen Bestimmungsmethoden für den organisch gebundenen Kohlenstoff (TOC) auf der Umwandlung dieses Kohlenstoffs in gasförmiges Kohlendioxid (CO₂) durch Oxidation. Entsprechende Verfahren sind beispielsweise aus der EP 1146335 B1, EP 0 663 239 B1, der WO 00/75653 A2, der EP 1 605 260 A2, der WO 99/42824 A1, der DE 1 966 760 A1, der DE 1 018 784 C2, der DE 10 121 999 A1 und der DE 253 462 A1 bekannt.

Die Oxidation erfolgt bei den bisher bekannten Verfahren entweder nasschemisch unter Zusatz starker Oxidationsmittel oder durch katalytische Hochtemperaturverbrennung mit Sauerstoff. Nach der Oxidation wird das gasförmige Kohlendioxid von der Probe separiert und analytisch bestimmt. Die Menge an gemessenem Kohlendioxid ist so ein direktes Maß für den Gehalt an organisch gebundenem Kohlenstoff der Probe.

Um die Bestimmung durchführen zu können, muss regelmäßig zuvor der anorganische Kohlenstoff (TIC), d. h. beispielsweise gelöstes Kohlendioxid, Kohlensäure und deren Salze, z.B. Carbonate und Hydrogencarbonate aus der Probe entfernt werden. So wird beispielsweise in der DE 19906151 A1, der DE 19616760 A1, der DE 4307814 A1, der DE 10018784 C2, der DE 1012199 A1, der EP 0663239 B1 sowie der WO 00/75653 beschrieben, dass die anorganischen Kohlenstoffverbindungen durch Ansäuern und anschließendes Austreiben entfernt werden können. Durch das Ansäuern werden in der Regel die Carbonate und Hydrogencarbonate in Kohlendioxid überführt. Dessen Austreibung kann z.B. durch Hitze, mittels Trägergasstroms oder mechanische Methoden erreicht werden. Beispielsweise wird in der DE 102004028270 B4 (US 2005/0282286 A1) und der EP 05104443 beschrieben, dass durch zusätzliche Schüttelbewegungen Vorgänge beschleunigt werden, die dazu beitragen, dass das Kohlendioxid schnell aus dem Reaktionsbereich mit der angesäuerten Probe in die Umgebung abgegeben wird.

Im Anschluss an die Entfernung des anorganisch gebundenen Kohlenstoffs erfolgt die Bestimmung des organisch gebundenen Kohlenstoffs durch Oxidation der Probe. Die Bestimmung des organisch gebundenen Kohlenstoffs ist demgemäss in der Regel ein zweistufiges Verfahren, welches die Entfernung des TIC und die anschließende Oxidation von organischem Kohlenstoff zur Bestimmung des TOC erfordert. Alternativ kann auch die sogenannte Differenzmethode zum Einsatz kommen. D.h., es werden Gesamtkohlenstoff- und anorganischer Kohlenstoffgehalt gemessen und aus der Differenz der beiden Messungen der Gehalt an organischem Kohlenstoff berechnet.

Für eine qualitativ hochwertige TOC-Analyse nach der Austreibmethode ist es somit generell entscheidend, dass
1. der TIC aus der Probe vollständig ausgetrieben wird,
2. beim TIC-Austreiben möglichst kein organischer Kohlenstoff verloren geht,
3. der verbliebene TOC komplett oxidiert und in Kohlendioxid überführt wird und
4. das gebildete Kohlendioxid quantitativ erfasst und detektiert wird.

Für die Durchführung solcher Verfahren werden nach dem Stand der Technik verschiedene Testkits beschrieben, welche in der Regel einen Reaktionsbereich und einen Detektionsbereich umfassen. D.h., in solchen Testkits wird zunächst die Probe durch Ansäuern von anorganischem Kohlenstoff befreit. Anschließend wird im Reaktionsbereich der organisch gebundene Kohlenstoff in gasförmiges Kohlendioxid umgewandelt und in den Detektionsbereich überführt. In diesem Detektionsbereich sind in der Regel Indikatoren vorhanden, welche infolge des Einflusses des Kohlendioxids eine Farbveränderung erfahren. Diese Farbveränderung ist das Maß für den Kohlendioxidgehalt. Solche Verfahren sind beispielsweise in der DE 10018784 und EP 0663239 A2 beschrieben.

Die mit den verschiedenen nach dem Stand der Technik bekannten Verfahren ermittelten Messwerte stimmen je nach Situation regelmäßig nicht exakt überein. Dies gilt beispielsweise für die unterschiedlichen Methoden der Entfernung des anorganischen Kohlenstoffs. So wird dies bei einigen Verfahren durch Ausblasen mit Stickstoff in stark saurer Lösung erreicht. Insbesondere bei solchen Verfahren besteht die Gefahr, dass organisch gebundener Kohlenstoff in Form von flüchtigen Kohlenwasserstoffen mit entfernt wird. In Abhängigkeit von dem eingesetzten Trägergasstrom, der Dauer des Durchflusses und der Form der Kohlenstoffverbindungen in der Probe kann eine erhebliche Verlustrate an organischem Kohlenstoff die Folge sein.

Ferner besteht bei Einstellung sehr niedriger pH-Werte, d.h. von Werten im Bereich pH 2 und darunter die Gefahr, dass Huminsäuren ausgefällt werden. Hierdurch werden unter Umständen auch Carbonate maskiert, verbleiben über die Austreibzeit in der Probe und führen in der Folge zu Mehrbefunden im Test.

Die Oxidation wird im Allgemeinen als am effektivsten angesehen, wenn diese in katalytischen Hochtemperaturverbrennungen mit Sauerstoff ausgeführt wird. Bei den nasschemischen Oxidationsverfahren wird in der Regel Peroxodisulfat als Oxidationsmittel eingesetzt. Ergänzt wird der Oxidationsprozess manchmal durch eine UV-Bestrahlung. Flankierend kann eine Optimierung der Temperatur und Reaktionszeit und des pH-Werts oder der Zusatz von Katalysatoren erfolgen, um die Oxidationsausbeute zu erhöhen. Denn bei den bisherigen nasschemischen Verfahren ist nicht von vornherein sichergestellt, dass die Oxidation quantitativ erfolgt und in der Folge die tatsächliche Menge an organisch gebundenen Kohlenstoff gemessen wird.

Aufgabe der vorliegenden Erfindung ist es demgemäss, einen Testkit zur Bestimmung des organisch gebundenen Kohlenstoffs in mit Partikeln belasteten Proben durch nasschemische Oxidation für den anschließenden Nachweis des entstandenen Kohlendioxids zur Verfügung zu stellen. Es soll erreicht werden, dass das Austreiben des anorganisch gebundenen Kohlenstoffs und die Oxidation des organisch gebundenen Kohlenstoffs zu Kohlendioxid mit einer einzigen Reaktionslösung durchführbar ist. Ferner sollen die oben beschriebenen Nachteile des bisherigen Standes der Technik vermieden werden, insbesondere soll die Ausfällung von Huminsäuren unterbunden werden.

Diese Aufgabe wird durch ein gelöst.

In einer bevorzugten Ausführungsform liegt der pH-Wert zwischen 2,7 und 3,3. Ganz besonders bevorzugt ist ein pH-Wert von ca. 3.

Die wässrige Lösung weist eine hohe Pufferkapazität auf. Unter Pufferkapazität ist erfindungsgemäß die Stärke einer Pufflösung zu verstehen, also das Ausmaß der Fähigkeit einer Elektrolytlösung, ihren pH-Wert bei Zusatz von Säuren oder Basen konstant zu halten. Mit hoher Pufferkapazität wird erfindungsgemäß eine Pufferstärke definiert, die bewirkt, dass bei Zugabe von 1 mol/I Protonen, beispielsweise durch Zusatz starker Mineralsäuren, der pH-Wert der Lösung um maximal 1 erniedrigt wird.

In einer erfindungsgemäßen Variante werden saure Puffer eingesetzt. Unter solchen sauren Puffern ist eine Substanz zu verstehen, deren wässrige Lösung einen pH-Wert von unter 7 aufweist und welche die pH-Wert-verändernde Wirkung zugesetzter Säuren oder Basen im Vergleich zu Wasser abmildert, also eine Pufferkapazität im oben definierten Sinne aufweist.

Als Puffer sind übliche, geeignete, dem Fachmann bekannte Säuren einsetzbar. Vorzugsweise werden erfindungsgemäß Phosphorsäure und deren Salze eingesetzt. Ebenso kann auch eine Kombination von Säure und Salz verwendet werden.

Die beschriebene wässrige Lösung weist somit einen pH-Wert von über 2 und höchstens 3,5 und vorzugsweise eine hohe Pufferkapazität auf. Dadurch ist die Probe zum einen stark genug angesäuert, um Carbonat quantitativ in Kohlendioxid zu überführen, aber zum anderen nicht so stark sauer, dass die darin enthaltenen Huminsäuren ausfallen. Infolge der hohen Pufferkapazität wird der pH-Wert während der Oxidationsreaktion ausreichend stabilisiert und dadurch etwaig anwesendes Chlorid weniger angegriffen, als dies im stark sauren Milieu der Fall ist.

Als Oxidationsmittel kommen verschiedene geeignete, dem Fachmann bekannte, Säuren und Salze in Betracht. Peroxidischwefelsäure (H₂S₂O₈), Periodsäure (H₅IO₆ bzw. HIO₄), Permangansäure (HMnO₄) sowie deren Salze oder Kombinationen einer oder mehrerer der genannten Säuren oder ihrer Salze. In einer erfindungemäß bevorzugten Variante werden Peroxodisulfat und Periodsäure kombiniert. Dadurch wird die Oxidationskraft gegenüber organischen Verbindungen und Partikeln deutlich erhöht.

Die Reagenzlösung kann Salze in einer derartigen Konzentration enthalten, dass die Lösung bei 20 °C in Bezug auf eines, mehrerer oder aller der Salze gesättigt ist. Als Salze kommen hier insbesondere die oben beschriebenen Verbindungen in Betracht. Daneben können für die Erreichung des Sättigungsgrades auch beliebige andere geeignete, dem Fachmann bekannte Salze zum Einsatz kommen. Durch die beschriebene Sättigung der Lösung ist eine lange Haltbarkeit des Oxidationsmittelgemisches gewährleistet. Durch die hohe Salzfracht des Puffers wird außerdem das Austreiben des anorganisch gebundenen Kohlenstoffs unterstützt.

Durch der Testkit wird eines Teils eine Austreibung des anorganisch gebundenen Kohlenstoffs erreicht und auf der anderen Seite der organisch gebundene Kohlenstoff zu Kohlendioxid oxidiert. Im Einzelnen gestaltet sich die Analysendurchführung wie folgt:
1. Die Probe wird in den Reaktionsbereich gegeben, der die Reagenzlösung bereits enthält. Hierdurch wird die Probe angesäuert und anorganischer Kohlenstoff in Kohlendioxid umgewandelt.
2. Durch heftige Bewegung des Reaktionsbereichs z.B. mit Hilfe der in der EP 05104443.6 A1 beschriebenen Vorrichtung, werden Probe und Reagenzlösung durchmischt und das Kohlendioxid aus dem Reaktionsbereich ausgetrieben.
3. Anschließend werden der Reaktions- und Detektionsbereich gasdicht miteinander verbunden. Dies kann z.B. mittels des in der EP 0663239 A2 beschriebenen Adapters erfolgen. Reaktions- und Detektionsbereich bestehen in diesem Beispiel aus zwei Glasküvetten, die an ihren offenen Enden mittels eines Adapters verschraubt werden.
4. Durch Erhitzen des Reaktionsbereichs wird die Oxidation des organisch gebundenen Kohlenstoffs zu Kohlendioxid erreicht und das Kohlendioxid in den Detektionsbereich übergetrieben.
5. Die Farbänderung der Indikatorlösung durch das übergetriebene Kohlendioxid wird gemessen. Als Messgerät eignet sich beispielsweise ein Photometer.

Durch entsprechende Maßnahmen ist zu gewährleisten, dass bei Verwendung des erfindungsgemäßen Testkits der anorganische Kohlenstoff im ersten Schritt zunächst alleine in Form von Kohlendioxid ausgetrieben wird, ohne dass zugleich eine Oxidation des organisch gebundenen Kohlenstoffs stattfindet. Im einfachsten Falle wird dies durch entsprechende Temperaturführung erreicht. D.h., zunächst wird dieReagenzlösung ohne Erwärmung der Probe eingesetzt. Im Anschluss daran erfolgt die Erhitzung, durch welche der Oxidationsvorgang des organisch gebundenen Kohlenstoffs hervorgerufen wird.

Alternativ kann der erfindungsgemäße Testkit zur Bestimmung des anorganischen Kohlenstoffs werden, indem auf die Erhitzung verzichtet wird.

Ebenso lässt sich der Testkit zur Bestimmung des Gesamtkohlenstoffs einsetzen. Damit eignet es sich auch für den Einsatz in der Differenzmethode, indem aus den ermittelten Gehalten am anorganischem Kohlenstoff und Gesamtkohlenstoff der Gehalt am organischen Kohlenstoff errechnet wird.

Im Folgenden wird die Erfindung unter Bezugnahme auf die Abbildungen näher beschrieben:

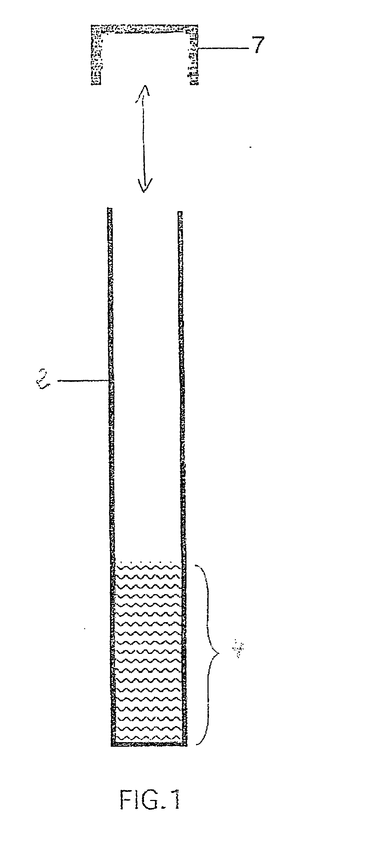

Fig. 1 zeigt ein Beispiel für einen einsetzbaren Reaktionsbereich des Testkits. Dieser besteht im Wesentlichen aus einem verschließbaren Behälter mit einer Verschlussvorrichtung 7, vorzugsweise einer Schraubverschlusskappe. Nach Abnahme der Kappe 7 wird die Probe in den Reaktionsbereich 2 gegeben. Dieser Reaktionsbereich enthält die vorzugsweise vorkonfektionierte Reagenzlösung. Nach Zusatz der Probe wird das freiwerdende anorganische Kohlendioxid mittels Bewegung ausgetrieben. Die Durchführung der Analyse kann in einer der im Folgenden beschriebenen Vorrichtungen durchgeführt werden.

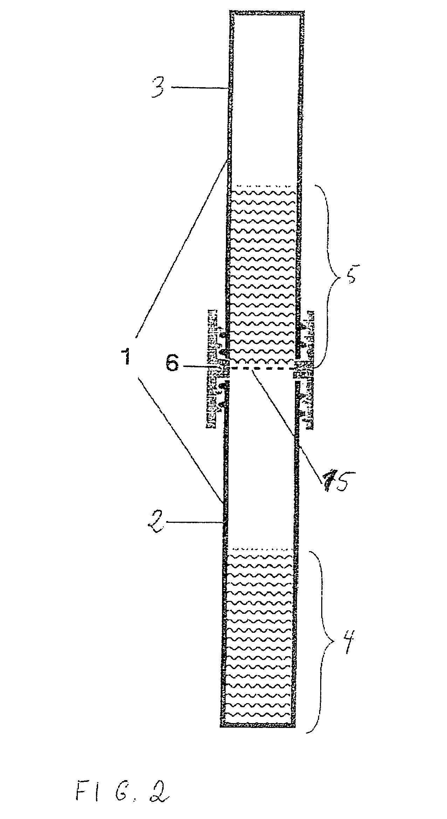

Fig. 2 zeigt ein Beispiel einer Vorrichtung, wie sie aus der EP 0663239 A2 bekannt ist. Hier wird in Analogie zur Beschreibung zur Figur 1 nach Zusatz der Reagenzlösung und der Probe in den Reaktionsbereich 2 das aus dem anorganisch gebundenen Kohlenstoff freigesetzte Kohlendioxid mittels Bewegung ausgetrieben. Danach wird der Reaktionsbereich 2 mit dem Detektionsbereich 3 verbunden. Im Reaktionsbereich 2 befindet sind die Reagenzlösung und im Detektionsbereich 3 eine Indikatorlösung 5. Die Verbindung der beiden Bereiche erfolgt in dem genannten Beispiel über einen Adapter 6, welche eine Membran 15 aufweist. Für die Erzeugung der gasförmigen Bestandteile aus der Probe 4 können neben dem Zusatz der Reagenzlösung noch flankierende Maßnahmen ergriffen werden. Als physikalische Maßnahme kommt ein Erhitzen in Betracht. Der chemisch gebundene organische Kohlenstoff wird durch die Reagenzlösung in Kohlendioxid umgewandelt und dieses Gas in die Indikatorlösung 5 übergetrieben. Der Reaktionsbereich 2 wird abgekühlt. Die Farbänderung der Indikatorlösung durch das übergetriebene Kohlendioxid wird in einem Photometer als Extinktion gemessen. Aus der Extinktion wird mittels Kalibrierdaten der TOC berechnet.

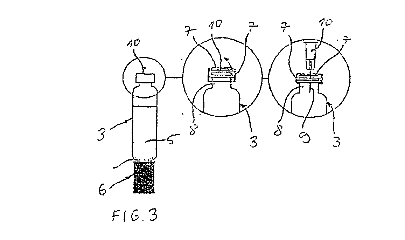

Die Figur 3 zeigt einen Testkit, wie er aus der DE 10018784 C2 bekannt ist. Im Unterschied zu dem Testkit gemäß Fig. 2 ist hier eine Verbindung des Detektionsbereichs 3 mit der Außenatmosphäre vorgesehen. Das über den Adapter 6 in die Indikatorlösung 5 des Reaktionsgefäßes 2 eingetriebene Gas wird wie in dem Beispiel gem. Fig. 2 beschrieben gemessen. Mittels der Nadel 10 kann der auf die Öffnung 8 aufgesetzte Verschluss nach außen hin geöffnet werden, um so über die Kanüle 9 eine Druckentlastung zu erzeugen. Dies kann zu einer zusätzlichen Beschleunigung des Gastransports beitragen. Hier wird, in Analogie zur Beschreibung in Fig. 1, nach Zusatz der Säuren und der Probe in den Reaktionsbereich, der analog zu Fig. 2 ausgeprägt ist, das freiwerdende Kohlendioxid erfindungsgemäß mittels Bewegung ausgetrieben.

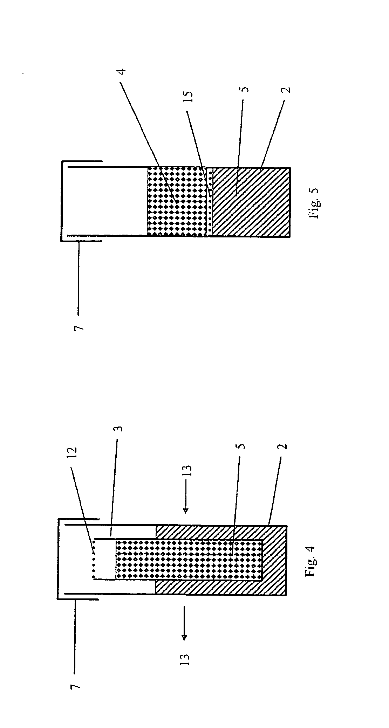

Figur 4 zeigt eine Vorrichtung, wie sie aus der WO 00/75653 bekannt ist. In diesem Beispiel ist in den Reaktionsbereich 2 der Detektionsbereich 3 mit der Indikationslösung 5 eingesetzt. Über den Gasraum sind die Gefäße miteinander verbunden. Hier wird in Analogie zur Beschreibung zu Figur 1 nach Zusatz der Reagenzlösung und der Probe in den Reaktionsbereich 2 das freiwerdende Kohlendioxid mittels Bewegung ausgetrieben. Durch Erhitzen wird das aus den gebundenen organischen Kohlenstoffen erzeugte Kohlendioxid aus der Probe befördert und mit dem Indikator 5 in Kontakt gebracht. Die Änderung des Indikators wird mittels Transmission eines Lichtstrahls 13 gemessen. In einer besonderen Ausführungsform können Reaktions- und Detektionsbereich über eine Membran miteinander verbunden sein.
Figur 5 zeigt ein Beispiel für einen Testkit, wie er aus der DE 10121999 A1 bekannt ist. Die Indikatorlösung 5 ist durch eine Membran 15 von der Reaktionslösung 4 getrennt. Über der Reaktionslösung 4 befindet sich ein Gasraum 14 und die Verschlusskappe 7. Zur Bestimmung des TOC wird die Küvette mit ihrem Verschlussdeckel 7 geöffnet und in Analogie zur Beschreibung in Figur 1 nach Zusatz der Reagenzlösung und der Probe in den Reaktionsbereich 2 das freiwerdende Kohlendioxid mittels Bewegung ausgetrieben. Nach Verschließen mit dem Deckel 7 wird die Küvette umgedreht und in einen Thermostat eingesetzt, wo durch Erhitzen des Reaktionsbereichs 4 aus dem gebundenen organischen Kohlenstoffs Kohlendioxid erzeugt wird, welches aus der Probe befördert und mit dem Indikator 5 in Kontakt gebracht wird. Im Anschluss daran erfolgt eine Messung der Farbänderung des Indikators 5.

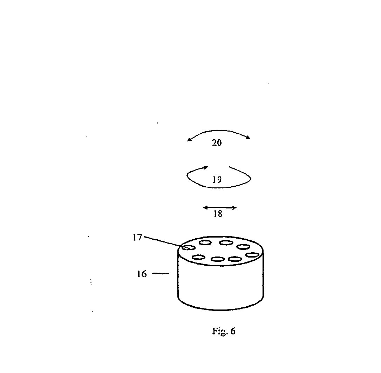

Figur 6 zeigt eine Variante für den Einsatz des erfindungsgemäßen Testkits. Neben dem Einsatz der Reagenzlösung zur Entfernung des anorganischen Kohlenstoffs ist als zusätzliche Maßnahme die Bewegung des Reaktionsbereichs 2 vorgesehen. Diese Bewegungen können in Form von Kreisbewegungen oder horizontalem Hin- und Herbewegen erfolgen. In der Variante gemäß Figur 6 kann zu diesem Zweck der Reaktionsbereich in die Vorrichtung 16 eingesetzt werden. In dieser Vorrichtung sind mehrere Öffnungen 17 vorgesehen. Hierdurch wird erreicht, dass eine Vielzahl von Reaktionsbereichen zugleich eingesetzt werden kann. Durch Bewegen in der Horizontale 18 erfolgt eine Durchmischung der Reaktionslösung mit der Reagenzlösung . In dem Beispiel gemäß Figur 6 ist auch eine bevorzugte Kreisbewegung vorgesehen. Ebenso möglich sind aber auch Kippbewegungen 20 oder Taumelbewegungen, sowie Kombinationen aller Bewegungsarten.

## Patentansprüche

1. Testkit zur Bestimmung des organisch gebundenen Kohlenstoffs in einer mit Partikeln belasteten Probe durch nass-chemische Oxidation und anschließenden Nachweis von Kohlendioxid enthaltend eine wässrige Reagenzlösung die
- einen pH-Wert von 2 bis 3,5 aufweist,
- ein Gemisch verschiedener Oxidationsmittel enthält, wobei als Oxidationsmittel Peroxodischwefelsäure, Periodsäure, Permangansäure, sowie deren Salze eingesetzt werden,
- eine Pufferkapazität aufweist, die bewirkt, dass bei Zugabe von 1 Mol Protonen pro Liter der Lösung deren pH-Wert um maximal 1 erniedrigt wird.

2. Testkit nach Anspruch 1, **dadurch gekennzeichnet, dass** das Reagenz einen pH-Wert zwischen 2,3 und 3,3 aufweist.

3. Testkit nach einem der vorhergehenden Ansprüche, **dadurch gekennzeichnet, dass** das Reagenz einen pH-Wert von etwa 3 aufweist.

4. Testkit nach einem der vorhergehenden Ansprüche, **dadurch gekennzeichnet, dass** das Reagenz einen sauren Puffer enthält.

5. Testkit nach Anspruch 4, **dadurch gekennzeichnet, dass** das Reagenz als Puffer Phosphorsäure, Phosphorsäuresalze oder eine Kombination hiervon enthält.

6. Testkit nach Anspruch 5, **dadurch gekennzeichnet, dass** das Reagenz Salze in einer derartigen Konzentration enthält, dass die Lösung bei 20°C in Bezug auf eines, mehrerer oder alle der Salze gesättigt ist.

7. Verfahren zur Bestimmung des organisch gebundenen Kohlenstoffs in einer mit Partikeln belasteten Probe, **dadurch gekennzeichnet, dass** remit einem Testkit nach einem der Ansprüche 1 bis 6 aus der Probe durch Kontakt mit dem Reagenz, anorganischer Kohlenstoff als Kohlendioxid freigesetzt oder der organisch gebundene Kohlenstoff zu Kohlendioxid oxidiert oder eine Kombination hiervon durchgeführt wird.

8. Verfahren nach Anspruch 7, **dadurch gekennzeichnet, dass** zunächst der anorganische Kohlenstoff und anschließend der organische Kohlenstoff in Form von Kohlendioxid ausgetrieben werden.

9. Verfahren nach Anspruch 8, **dadurch gekennzeichnet, dass** aus den Kohlenstoffverbindungen durch nass-chemische Reaktion Kohlendioxid gebildet wird.

10. Verfahren nach Anspruch 9, **dadurch gekennzeichnet, dass** die Menge des aus dem gebundenen Kohlenstoff gebildeten Kohlendioxids durch Farbänderung eines Indikators mit an sich bekannten Messverfahren bestimmt wird.

11. Verwendung des Testkits nach einem der Ansprüche 1 bis 6 zur Bestimmung des organisch gebundenen Kohlenstoffs in einer mit Partikeln belastetean Probe.

## Claims

1. Test kit for determining the organically bound carbon in a particle-polluted sample by wet-chemical oxidation and subsequent detection of carbon dioxide containing an aqueous reagent solution which
- has a pH of 2 to 3.5,
- contains a mixture of various oxidizing agents, wherein the oxidizing agents used are peroxo-disulphuric acid, periodic acid, permanganic acid, and also salts thereof,
- has a buffer capacity which has the effect that, on addition of 1 mol of protons per litre of the solution its pH is decreased by a maximum of 1.

2. Test kit according to Claim 1, **characterized in that** the reagent has a pH between 2.3 and 3.3.

3. Test kit according to any of the preceding claims, **characterized in that** the reagent has a pH of about 3.

4. Test kit according to any of the preceding claims, **characterized in that** the reagent contains an acidic buffer.

5. Test kit according to Claim 4, **characterized in that** the reagent contains, as buffer, phosphoric acid, salts of phosphoric acid or a combination thereof.

6. Test kit according to Claim 5, **characterized in that** the reagent contains salts in a concentration such that the solution at 20°C is saturated with respect to one, a plurality, or all of the salts.

7. Method of determining the organically bound carbon in a particle-polluted sample, **characterized in that** using a test kit according to any of Claims 1 to 6, from the sample, using the reagent, inorganic carbon is released as carbon dioxide or the organically bound carbon is oxidized to carbon dioxide, or a combination thereof is carried out.

8. Method according to Claim 7, **characterized in that** first the inorganic carbon and subsequently the organic carbon is expelled in the form of carbon dioxide.

9. Method according to Claim 8, **characterized in that** carbon dioxide is formed from the carbon compounds by wet-chemical reaction.

10. Method according to Claim 9, **characterized in that** the amount of the carbon dioxide formed from the bound carbon is determined by colour change of an indicator using measuring methods known per se.

11. Use of the test kit according to any of Claims 1 to 6 for determining the organically bound carbon in particle-polluted samples.

## Revendications

1. Nécessaire d'essai pour la détermination du carbone en liaison organique dans un échantillon chargé de particules, par oxydation chimique en humide et détermination subséquente du dioxide de carbone contenant une solution aqueuse de réactif, qui
- présente un pH de 2 à 3,5,
- contient un mélange de divers oxydants, l'acide peroxodisulfurique, l'acide periodique, l'acide permanganique ainsi que leurs sels étant utilisés comme oxydants,
- présente un pouvoir tampon qui agit de sorte que lors d'addition de 1 mole de protons par litre de la solution, son pH est abaissé de 1 au maximum.

2. Nécessaire d'essai selon la revendication 1, **caractérisé en ce que** le réactif présente un pH compris entre 2,3 et 3,3.

3. Nécessaire d'essai selon l'une quelconque des revendications précédentes, **caractérisé en ce que** le réactif présente un pH d'environ 3.

4. Nécessaire d'essai selon l'une quelconque des revendications précédentes, **caractérisé en ce que** le réactif contient un tampon acide.

5. Nécessaire d'essai selon la revendication 4, **caractérisé en ce que** le réactif contient en tant que tampon de l'acide phosphorique, des sels d'acide phosphorique ou une association de ceux-ci.

6. Nécessaire d'essai selon la revendication 5, **caractérisé en ce que** le réactif contient des sels en une association telle que la solution est saturée à 20 °C eu égard à un, plusieurs ou la totalité des sels.

7. Procédé pour la détermination du carbone en liaison organique dans un échantillon chargé de particules, **caractérisé en ce qu'**à l'aide d'un nécessaire d'essai selon l'une quelconque des revendications 1 à 6, par contact avec le réactif du carbone inorganique est libéré sous forme de dioxyde de carbone à partir de l'échantillon, ou le carbone en liaison organique est oxydé en dioxyde de carbone ou une combinaison de ces processus est effectuée.

8. Procédé selon la revendication 7, **caractérisé en ce que** d'abord le carbone inorganique et ensuite le carbone organique sont expulsés sous forme de dioxyde de carbone.

9. Procédé selon la revendication 8, **caractérisé en ce que** du dioxyde de carbone est formé par réaction chimique en humide à partir des composés carbonés.

10. Procédé selon la revendication 9, **caractérisé en ce que** la quantité du dioxyde de carbone formé à partir du carbone lié est déterminée, à l'aide de procédés de mesure connus en soi, par changement de couleur d'un indicateur.

11. Utilisation du nécessaire d'essai selon l'une quelconque des revendications 1 à 6, pour la détermination du carbone en liaison organique dans un échantillon chargé de particules.
